# EUROPEAN PATENT SPECIFICATION

(11) **EP 0 419 559 B1**
(45) Date of publication and mention of the grant of the patent: **30.08.1995**
(21) Application number: 89907536.0
(22) Date of filing: 07.06.1989
(51) Int. Cl.: A61C 19/06

(54) **TOOTH-ANCHORED BENEFICIAL AGENT DELIVERY DEVICE**
AM ZAHN BEFESTIGTE VERABREICHUNGSANORDNUNG EINES HEILSAMEN MITTELS
DISPOSITIF D'ADMINISTRATION D'AGENT BENEFIQUE FIXE A UNE DENT

(30) Priority: 13.06.1988 US 206098; 14.04.1989 US 338057
(43) Date of publication of application: 03.04.1991
(73) Proprietor: TRANSPHARM GROUP, San Francisco, CA 94117 (US)
(72) Inventor: GARAY, Gabriel, L., San Francisco, CA 94117 (US); GARAY, Anne-Ly, Redwood City, CA 94062 (US); TACY, Robert, Los Altos Hills, CA 94022 (US)
(74) Representative: Goldin, Douglas Michael
(86) International application number: US8902488
(87) International publication number: WO8912430

(56) References cited:
- US-A- 0 307 537
- US-A- 2 378 279
- US-A- 2 835 628
- US-A- 3 429 308
- US-A- 4 020 558
- US-A- 4 175 326
- US-A- 4 681 544
- US-A- 4 741 700

## Description

### Technical Field

The invention is in the field of drug delivery. More specifically it is in the field of sustained-release devices for administering drugs or other beneficial agents orally.

### Background

The prolonged delivery of drugs orally has been a major challenge and a long desired objective in drug therapy. This is a favored route for drug administration. It is estimated that 65% of all drugs are ingested. The successful accomplishment of prolonged oral drug delivery has great therapeutic significance in the treatment of various diseases and conditions.

Systemic and transdermal sustained drug delivery systems have been developed which are capable of delivering constant amounts of therapeutic substances from several days to several months. The major limitation to long-term oral delivery, however, is the 8-16 hour gastrointestinal transit time of an ingested substance. In order to achieve uninterrupted action for longer than 24 hours by a therapeutic substance, its passage needs to be slowed in the gastrointestinal tract or the delivery device supplying the drug has to be fixed or immobilized within the tract.

Attempts have been mode either to incorporate drugs into floating devices which would empty less readily from the stomach (N. Eng. J. Med. (1981) 304:1365-1366) or to utilize insoluble bioadhesive polymers as carriers for drugs. It is thought that a delivery device made of such a polymer would adhere to the mucosa of the gastrointestinal tract and be able to discharge its contents in a sustained manner ("Advances in Drug Delivery Systems" J.M. Anderson and S.W. Kim, Eds, Elsevier, Amsterdam Vol 1, 1986, pp. 47-57). Although some of these approaches have had limited success in animals, they have proved to be impractical in humans thus far.

The diseases and conditions of dentition and of the oral cavity, on the other hand, have been more freely targeted for timed-release chemicals or therapeutic substances which have been placed inside delivery devices intended to act for longer than 24 hours. Considerable attention has been paid to the control of bad breath or taste in the mouth by means of dispensing deodorizers or chemicals that impart a pleasant taste or mask unpleasant odors. U.S. 4,741,700 describes a breath-freshening device that comprises a cup-shaped base that is bonded to a tooth and a saliva-soluble tablet-shaped mint that fits into the base. Alternatively, the mint can be in the form of a plurality of pills adherent to the surface of the base (Fig. 4) or a toroid that fits over a port on the base (Fig. 6). While this patent suggests that the breath-freshening mint could be replaced with medicament, there is no provision that would prevent the tablet, pills, or toroid from being inadvertently or unintentionally dislodged from the base. The possibility of such dislodgment makes the suitability of the device for sustained drug delivery questionable.

U.S. Patent 2,378,279 discloses an orthodontic band clamp which is placed about a tooth and may be adjusted in a variety of ways to cling to a tooth and support various dental appliances.

U.S. Patents 3,503,127 and 3,600,807 describe inventions where a cup or pocket is formed in a denture or in an artificial tooth in order to store and make available breath- and taste-refreshing chemicals. U.S. Patent 2,835,628 proposes the use of medicated tape impregnated with prolonged-release sodium fluoride. This device is made to adhere to the tooth's surface for the prevention of dental caries. In another invention a soluble tape impregnated with fluoride or other chemicals for the local treatment of oral diseases is inserted between teeth and left there to dissolve and disperse its active ingredient (U.S. Patent 3,754,332).

Other inventions propose to employ biocompatible adhesives and patches in order to affix tablets directly to the buccal mucosa for odor masking, for buccal delivery of local anesthetics and antihistamines, or even for the local release of nitroglycerin (U.S. Patents 3,911,099 and 4,039,653). However, most of these delivery systems are expected to remain fixed only for a relatively short period of time.

U.S. Patent 3,429,308 also discloses a device for administering a beneficial agent in the oral cavity, which is held in direct contact with the buccal mucosa. The device utilises one or a strip of lozenges of gelatinous material, which act as a reservoir for one or more drugs. The device is designed to adhere to the inner lip by suction. In use, such a device also suffers from the disadvantage that it is readily dislodged from its initial site of adherence.

French Patent 2,278,317 and corresponding U.S. Patent 4,020,558 describe what is termed a "buccal implant" having at least one medicated tab attached to a blunt-nosed spike which can be pushed into the space between two adjacent teeth. The objective is to provide a local, sustained release of medication(s) when the tab comes into contact with the gum line. Such a device may, however, be harmful if it unintentionally becomes dislodged and ingested or aspirated, particularly in view of the spike.

The use of a medicated pad placed over the open socket of a freshly extracted tooth for the relief of pain and haemorrhage was also suggested (U.S. 3,386,440). There is significant interest in treating gingivitis and periodontal diseased by placing long-acting medications locally in the vicinity of the inflamed gum or by introducing active substances directly into the disease-induced subgingival pockets (J. Periodontology (1984) 11:651-651).

Goodson (U.S. Patent 4,175,326) discloses capillary hollow fibres filled with the antibiotic tetracycline. These hollow fiber bands are slipped over each tooth, then rolled down into the periodontal pocket in order to achieve a high local concentration of the antibiotic. The control of the local microbial flora linked to the pathogenesis of this disease is the aim of this process.

The further utilization of drug-loaded hollow fiber bands for the therapy of other oral conditions requiring the local availability of anti-inflammatory, antifungal, or immune modulator substances is also suggested. Jernberg in U.S. Patent 4,685,883 describes a delivery system of biodegradable, time-release micro-spheres encapsulating drugs and packed tightly into the subgingival periodontal pocket. He also suggests the use of a biodegradable matrix adhesively attached to the root of the tooth for the more effective local treatment of the periodontal disease is also suggested.

U.S. Patent 4,681,544 describes an oral pack retention system to hold surgical dressings over a fresh wound site in order to prevent infection and hemorrhage after oral or periodontal surgery. The pack may be impregnated with drugs such as antibiotics or analgesics. The patent extends the invention of Goodson and suggests that its system, using wires and adhesives, could retain a medicated pack at the root of a tooth for the local treatment of periodontal disease. A complex and extensive system of individually adjustable splints and wires to support the pack is described.

Since most of these oral, active-substance delivery systems target the local therapy of oral diseases, they have significant limitations as to the amount of drug which can be placed, as well as limitations in the amount of time the devices will remain in place. Furthermore, there can be difficulties when these devices need to be removed, once their active content has been discharged. Replacement for repeated administration and means for preventing unintentional dislodgment have been particularly ignored.

The prior art is particularly devoid of devices which are designed for the sustained delivery of drugs and/or other beneficial substances for the whole body, rather than just the oral cavity. There is a need for devices which are reliably held within the mouth, can be repeatedly replenished with active substances, are easily tolerated in the mouth, and do not interfere with speech, food mastication or oral hygiene. The present invention provides such devices.

In one aspect, the present invention provides a beneficial agent delivery device adapted to be semipermanently affixed to a tooth comprising:
(a) an anchor member that is adapted to be removably affixed to the side of a tooth out of the way of the biting surface of the teeth; and
(b) a cartridge member adapted to be removably and interlockingly affixed to said anchor member and containing a beneficial agent which is capable of release to the oral cavity, characterised in that said cartridge member comprises a chain of individual cartridges that are linked to each other.

The invention thus provides a tooth-borne platform that may be worn comfortably and that mechanically interlocks with a removable cartridge from which drugs or other beneficial agents may be released into the oral cavity and gastrointestinal tract at pre-determined doses and dosage regimes. The interlocking feature of the cartridge prevents inadvertent dislodgment of drug into the mouth which could result in premature swallowing of the drug and noncompliance with a sustained administration regime. The provision of an agent-carrying means having a multiplicity of linked individual cartridges facilitates tailoring of the dose, and/or dosage regime or the administration of a multiplicity of agents simultaneously or in a predetermined sequence. Further, since the agent-carrying means is removable, it may be easily removed and, if necessary, replaced immediately or after a predetermined interval. Since the device permits reliable sustained release it facilitates patient compliance.

Further the mounting of the invention device is a nonsurgical, painless event. The device, when properly worn, should not inhibit normal physiological functions or cause significant irritation to oral tissues.

### Brief Description of the Drawings

In the drawings, which are not to scale and in which like parts are referred to by the same reference numeral:
Fig. 1 is a view of a lower dentition showing an embodiment of the invention device anchored to a tooth.
Fig. 2 is an enlarged exploded view of the device of Figure 1.
Fig. 3 is a cross-sectional view of the device of Figure 1.
Fig. 4 is a plan view of one of the removable elements of the device of Figure 1.
Fig. 5 depicts another embodiment of the invention device.
Fig. 6 is a cross-sectional view of yet another embodiment of the invention device.
Fig. 7 is an exploded view of the device of Figure 6.
Fig. 8 is a partial view of a human head showing the device of Fig. 1 anchored to a tooth and the anatomical position of the device.

### Detailed Description of Embodiments Shown in Drawings

The term "beneficial agents" as used herein is intended to include drugs, vitamins and other compositions that are administered orally to humans or animals to achieve a beneficial effect on the recipient.

The term "drug" as used herein broadly includes physiologically and/or pharmacologically active substances for producing a local effect within the mouth or gastro-intestinal tract or a systemic effect at a remote site within the body.

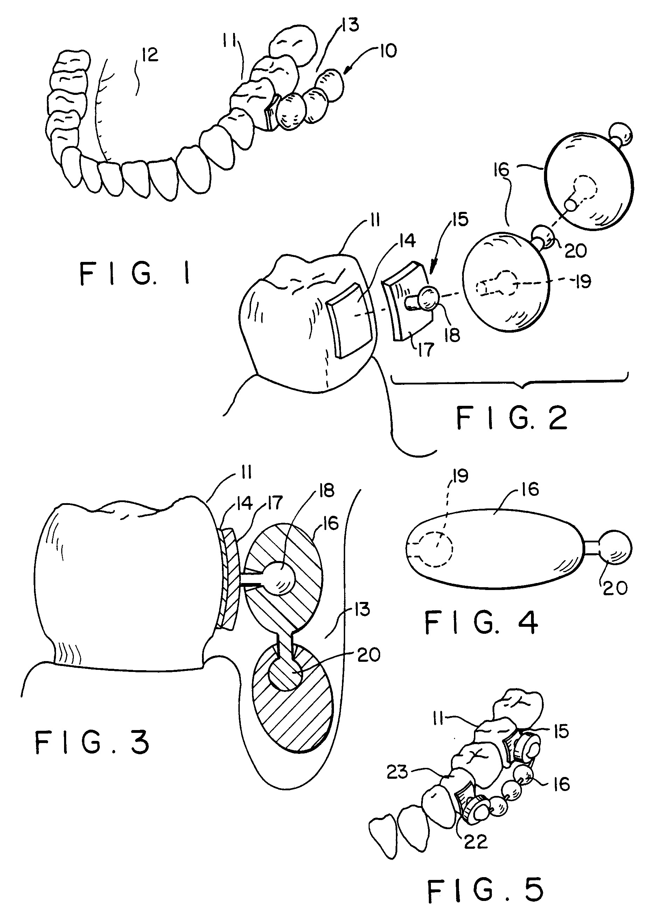

Figs. 1-4 and 8 depict one embodiment of the invention device, generally designated 10, anchored to a tooth 11 and show the position of the device relative to the tongue 12 and cheek vestibule 13. As shown, in Figs. 1 and 8 device 10 lies buccally within the vestibule of the cheek/lips out of the way of the biting surface of the teeth. While the device is shown in a buccal position, it could, if desired, be positioned on the lingual side of the teeth. Further, the device may be mounted in either the upper or lower dentition.

Referring to Fig. 2, the device includes an adhesive layer 14 one face of which is adhered to the labial or buccal (outer) side of the tooth, an anchor member 15, and a multiplicity of cartridges 16. The anchor member 15 is semipermanently affixed (i.e., for at least a day and usually for at least a week) to the labial side of the tooth via the adhesive layer and consists of a flat base 17 and a ball member 18 that extends outwardly from the flat base. The adhesive may be biodegradable or nondegradable, but in any event should be such as to permit the anchor member to be removed from the tooth when the delivery is no longer desired. Means other than adhesives such as bonding or mechanical devices may be used to affix the anchor member to the tooth. The cartridge capsule that is attached directly to the anchor member has a socket 19 formed in its surface that is adapted to receive ball member 18. This ball-and-socket arrangement provides an interlocking mechanism that requires positive patient or physician intervention to unlock or dislodge the cartridge from the anchor membrane. Alternatively, other equivalent interlocking connecting means such as key-and-slot arrangements for removably connecting the cartridge to the anchor may be used. The cartridge may thus be removably connected to the anchor member by the ball-and-socket combination. Ball-and-socket connection permits rotational movement of the cartridge about the ball-socket axis. When it is desired to have a multiplicity of individual cartridges removably connected to each other in chain-fashion and to the anchor member, the initial cartridge in the chain carries a ball member 20 on its exterior surface. That ball member is received in a socket formed in the second cartridge in the chain, and so forth. As shown the ball member and socket in the intermediate cartridges in the chain are positioned along the same axis. Alternatively, a branched assembly is possible by providing the cartridges with more than one inter-connection site. Accordingly, the cartridges are connected to the anchor in a manner that permits each cartridge to rotate, thereby imparting the chain with the ability to conform to the anatomy of the cheek/lip vestibule. Further, the chain is able to flex freely to avoid interfering with chewing or dental hygiene.

At least one of the cartridges, and preferably all of the cartridges contain a beneficial agent. By using a multiplicity of unit dosage cartridges, doses may be easily adjusted via the number of cartridges included in the chain. The individual cartridges may contain different agents. The structure of the cartridges and/or the formulation of the agent within the cartridges are preferably such that the release of agent from the cartridges will be over a sustained time period. It will be appreciated that the agent may be formulated in such a manner (e.g., in coated micro-capsules) that while the formulation may be released initially in the oral cavity that the release of agent from the formulation occurs elsewhere in the gastro-intestinal tract. Various mechanisms to achieve sustained release such as diffusion, osmosis, bioerosion, swelling, and dissolution may be employed. The cartridge may be of monolithic or container construction. Monolithic structures are composed entirely of drug formulation or drug dispersed in a matrix material. Container structures comprise a housing or wall that defines a lumen and an agent formulation within the lumen. In either structure, the cartridge is made of a material that permits the formation of the socket/ball member or other interconnecting means required to interconnect the cartridges. In container structures, depending on the nature of the agent release mechanism, the wall may also be required to be permeable to the agent or be semipermeable to permit aqueous fluids to be imbibed into the cartridge to effect agent release by an osmotic mechanism. Alternatively, the cartridge may be porous so as to permit saliva to enter the cartridges to contact the agent or agent-release mechanism within the cartridge. The agent within the cartridge may be neat or formulated with carriers or diluents, again depending upon the nature of the agent-release mechanism.

The shape of the cartridges 16 is generally not critical and will normally be chosen for ease of manufacture and comfortable residence within the cheek/lip vestibule. Shapes that are typically used for tablets, capsules, and pills are acceptable.

Figure 5 shows a variation of the device of Figure 1 in which a second anchor member 22 is attached to the outer site of another tooth 23 in the lower dentition and the cartridge at the end of the chain is removably connected to the second anchor member. The chain of cartridges is thus suspended between the two anchor members along the lower dentition. Such assembly assures that the chain is kept out of the way of the biting surfaces and is maintained in an extended configuration.

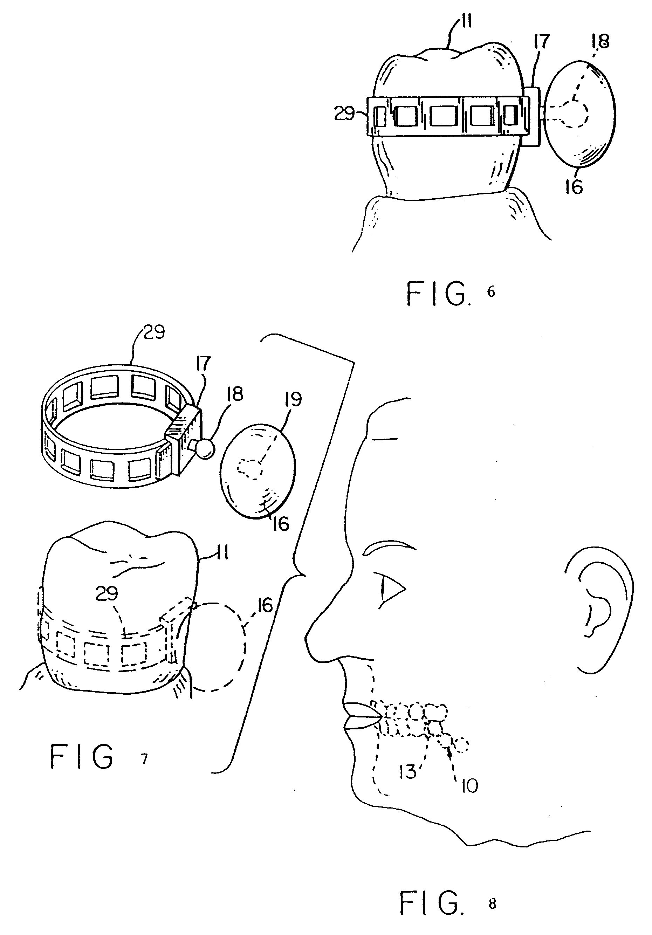

Figures 6 and 7 depict a variation of the device of Figure 1 in which a tooth-encircling band or "lasso" 29 is used instead of or in combination with an adhesive as a means for attaching the anchor member to a tooth. The band may be made of an elastic material, a rigid material, or may be mechanically adjustable (e.g., by a ratchet mechanism) so that its girth may be altered to encircle the tooth snugly. With elastic materials the band is self-adjusting to encircle the tooth snugly. In the case of rigid materials, the girth of the band is preset to provide a snug fit. The band may be further secured with an adhesive or cement. While the band is shown encircling a single tooth, it may encircle more than one tooth if desired.

Other embodiments of the invention that are obvious to those of skill in the fields of drug delivery devices, orthodontics, or related fields are intended to be within the scope of the following claims.

## Claims

1. A beneficial agent delivery device adapted to be semipermanently affixed to a tooth comprising:
(a) an anchor member (15) that is adapted to be removably affixed to the side of a tooth out of the way of the biting surface of the teeth; and
(b) a cartridge member adapted to be removably and interlockingly affixed to said anchor member and containing a beneficial agent which is capable of release to the oral cavity, characterised in that said cartridge member comprises a chain of individual cartridges (16) that are linked to each other.

2. The device of claim 1 including means for semipermanently affixing said anchor member to the tooth.

3. The device of claim 2 wherein said means is an adhesive or a bonding agent.

4. The device of claim 2 wherein the means is a band (29) that encircles the tooth and to which the anchor member (15) is attached.

5. The device of claim 4 wherein the girth of the band (29) is adjustable.

6. The device of claim 4 wherein the girth of the band (29) is preset to encircle the tooth snugly.

7. A device as claimed in any one of claims 1 to 6 wherein the anchor member comprises a base member (17) that is adapted to be affixed to the side of the tooth and a ball member (18) extending outwardly from the base member and the cartridge member has a socket (19) into which the ball member interlockingly fits.

8. A device as claimed in any one of claims 1 to 7 wherein the individual cartridges of said chain (16) are linked to each other by an interlocking ball-and-socket means.

9. A device as claimed in any one of claims 1 to 8 including a second anchor member (22) that is adapted to be affixed to the same side of another tooth out of the way of the biting surface of the teeth, and wherein one end of said chain is removably affixed to one anchor member (15) and the other end of the chain is removably affixed to the second anchor member (22).

## Patentansprüche

1. Vorrichtung zur Verabreichung eines heilsamen Mittels, die halbdauerhaft an einem Zahn befestigt werden kann, umfassend:
(a) ein Verankerungselement (15), das lösbar an der Seite eines Zahnes außer Reichweite der Bißfläche der Zähne befestigt werden kann; und
(b) ein Patronenelement, das lösbar und ineinandergreifend an dem Verankerungselement befestigt werden kann und ein heilsames Mittel enthält, das in die Mundhöhle freigesetzt werden kann, dadurch gekennzeichnet, daß das Patronenelement eine Kette einzelner Patronen (16) enthält, die miteinander verbunden sind.

2. Vorrichtung nach Anspruch 1, umfassend eine Einrichtung zur halbdauerhaften Befestigung des Verankerungselements an dem Zahn.

3. Vorrichtung nach Anspruch 2, bei der die Einrichtung ein Klebe- oder Haftmittel ist.

4. Vorrichtung nach Anspruch 2, bei der die Einrichtung ein Band (29) ist, das den Zahn umgibt, und an dem das Verankerungselement (15) befestigt ist.

5. Vorrichtung nach Anspruch 4, bei der der Umfang des Bandes (29) einstellbar ist.

6. Vorrichtung nach Anspruch 4, bei der der Umfang des Bandes (29) so voreingestellt ist, daß das Band den Zahn eng umschließt.

7. Vorrichtung nach einem der Ansprüche 1 bis 6, bei der das Verankerungselement ein Basiselement (17) umfaßt, das an der Seite des Zahnes befestigt werden kann, sowie ein Kugelelement (18), das von dem Basiselement nach außen ragt, und bei der das Patronenelement eine Fassung (19) besitzt, in die das Kugelelement in ineinandergreifender Weise paßt.

8. Vorrichtung nach einem der Ansprüche 1 bis 7, bei der die einzelnen Patronen der Kette (16) durch eine ineinandergreifende Kugelgelenkeinrichtung miteinander verbunden sind.

9. Vorrichtung nach einem der Ansprüche 1 bis 8, umfassend ein zweites Verankerungselement (22), das an derselben Seite eines anderen Zahnes außer Reichweite der Bißfläche der Zähne befestigt werden kann, und bei der ein Ende der Kette lösbar an einem Verankerungselement (15) befestigt ist und das andere Ende der Kette lösbar an dem zweiten Verankerungselement (22) befestigt ist.

## Revendications

1. Dispositif d'administration d'agent médicamenteux conçu pour être fixé d'une manière semi-permanente à une dent, comprenant:
(a) un élément d'accrochage (15) conçu pour être fixé d'une manière amovible sur le côté d'une dent sans empiéter sur la surface active des dents; et
(b) un élément formant cartouche conçu pour être fixé d'une manière amovible et verrouillable audit élément d'accrochage et contenant un agent médicamenteux qui peut être libéré dans la cavité buccale, caractérisé en ce que ledit élément formant cartouche comporte une chaîne de cartouches individuelles (16) reliées les unes aux autres.

2. Dispositif selon la revendication 1, comprenant un moyen pour fixer d'une manière semi-permanente ledit élément d'accrochage à la dent.

3. Dispositif selon la revendication 2, dans lequel ledit moyen est une colle ou un agent adhésif.

4. Dispositif selon la revendication 2, dans lequel ledit moyen est une bande (29) qui entoure la dent et à laquelle est fixée l'élément d'accrochage (15).

5. Dispositif selon la revendication 4, dans lequel la circonférence de la bande (29) est réglable.

6. Dispositif selon la revendication 4, dans lequel la circonférence de la bande (29) est préétablie pour entourer la dent sans trop la serrer.

7. Dispositif selon l'une quelconque des revendica-tions 1 à 6, dans lequel l'élément d'accrochage comporte une base (17) conçue pour être fixée sur le côté de la dent et une rotule (18) s'étendant vers l'extérieur depuis la base, et la cartouche a un évidement (19) dans lequel la rotule se loge en réalisant un verrouillage.

8. Dispositif selon l'une quelconque des revendica-tions 1 à 7, dans lequel les différentes cartouches de ladite chaîne (16) sont reliées les unes aux autres par un moyen de verrouillage à rotule.

9. Dispositif selon l'une quelconque des revendica-tions 1 à 8, comprenant un second élément d'accrochage (22) conçu pour être fixé au même côté d'une autre dent sans empiéter sur la surface active des dents, et dans lequel une première extrémité de ladite chaîne est fixée d'une manière amovible à un élément d'accrochage (15) et l'autre extrémité de la chaîne est fixée d'une manière amovible au second élément d'accrochage (22).
